(19) Europäisches Patentamt
European Patent Office
Office européen des brevets

(11) **EP 3 804 679 B1**

(12) **EUROPEAN PATENT SPECIFICATION**

(45) Date of publication and mention
of the grant of the patent:
**04.06.2025 Bulletin 2025/23**

(21) Application number: **19810814.4**

(22) Date of filing: **09.04.2019**

(51) International Patent Classification (IPC):
***A61F 13/514*** *(2006.01)*

(52) Cooperative Patent Classification (CPC):
**A61F 13/15699; A61F 13/4755; A61F 13/49446;**
**A61F 13/51478; A61F 13/537; B32B 5/022;**
**B32B 5/26; B32B 7/12; B32B 29/02;**
A61F 2013/15959; A61F 2013/15967;
A61F 2013/5149; B32B 2250/04; B32B 2250/05;
B32B 2262/0238; (Cont.)

(86) International application number:
**PCT/JP2019/015407**

(87) International publication number:
**WO 2019/230200 (05.12.2019 Gazette 2019/49)**

(54) **ABSORBENT ARTICLE**

ABSORBIERENDER ARTIKEL

ARTICLE ABSORBANT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **01.06.2018 JP 2018106250**
**08.03.2019 JP 2019042349**

(43) Date of publication of application:
**14.04.2021 Bulletin 2021/15**

(73) Proprietor: **Kao Corporation**
**Chuo-ku,**
**Tokyo 103-8210 (JP)**

(72) Inventors:
• **MAEDA, Naruaki**
**Haga-gun, Tochigi 321-3497 (JP)**
• **SASE, Masakazu**
**Haga-gun, Tochigi 321-3497 (JP)**

(74) Representative: **Vossius & Partner**
**Patentanwälte Rechtsanwälte mbB**
**Siebertstrasse 3**
**81675 München (DE)**

(56) References cited:
WO-A1-2004/094136        WO-A1-2013/099463
JP-A- 2013 013 641        JP-A- H11 290 381
US-A1- 2010 028 638

(52) Cooperative Patent Classification (CPC): (Cont.)
B32B 2262/0246; B32B 2262/0253;
B32B 2262/0261; B32B 2262/0284; B32B 2307/718;
B32B 2307/726; B32B 2307/7265; B32B 2307/728;
B32B 2555/02; D06C 11/00; D06C 23/04

**Description**

FIELD OF THE INVENTION

The present invention relates to an absorbent article, such as a sanitary napkin or a diaper.

BACKGROUND OF THE INVENTION

Nonwoven fabric is often used in an absorbent article such as a sanitary napkin from a viewpoint of texture or the like. Various proposals have been offered regarding such nonwoven fabrics.

For example, JP-A-2016-65335 describes a nonwoven fabric including raised fibers incorporated with free fibers intersecting the raised fibers from a viewpoint of improving texture of the nonwoven fabric.

JP-T-2011-529749 describes an absorbent article having a barrier layer containing a spunbond nonwoven fabric web and a meltblown nonwoven fabric web. From a viewpoint of providing the barrier layer with not only barrier properties but also high flexibility and softness for the sensitive skin, number average fiber diameter of each nonwoven fabric web and total weight percentage of the meltblown nonwoven fabric web are held within specific ranges.

WO 2004/094136 A1 discloses that the process of laminating meltbolown fibers to the surface of the spunbond web make meltbolown fibers enter into the long fiber layer of the spunbond web. JP 2013-13641 A discloses a discloses a water-repellent nonwoven fabric such as a general spunbond nonwoven fabric or a general SMS nonwoven fabric.

SUMMARY OF THE INVENTION

The present invention provides an absorbent article including a top layer, a leak-proof layer, and an absorbent layer disposed between the top layer and the leak-proof layer, as defined in independent claim 1. Preferre aspects are defined in the dependent claims.

In an embodiment, the leak-proof layer includes an embossed part in which the meltblown layer and the protective layer are bonded, and a non-embossed part other than the embossed part.

In an embodiment, the leak-proof layer includes a non-opening region in the non-embossed part.

In an embodiment, the non-opening region is a region which does not include a through-hole in which, in a plane view of the leak-proof layer, an interfiber space containing a region in which an area of a rectangle formed of 10 $\mu$m in a diagonal line length reaches 25 $\mu$m$^2$ or more passes therethrough in a thickness direction.

In an embodiment, the non-opening region is arranged on a region in which the leak-proof layer overlaps with the absorbent layer.

Other and further objects, features and advantages of the invention will appear more fully from the following description, appropriately referring to the accompanying drawings.

BRIEF DESCRIPTION OF DRAWINGS

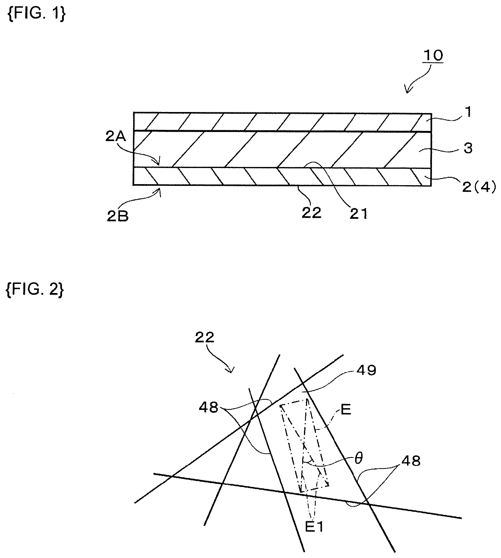

{FIG. 1}
FIG. 1 is a cross-sectional drawing showing one embodiment of an absorbent article according to the present invention.
{FIG. 2}
FIG. 2 is an explanatory drawing schematically showing a method for confirming a "region in which an area of a rectangle formed of 10 $\mu$m in a diagonal line length reaches 25 $\mu$m$^2$ or more" for a nonwoven fabric constituting a leak-proof layer of an absorbent article according to the present invention.
{FIG. 3}
FIG. 3 is a partially enlarged cross-sectional drawing schematically showing a preferable embodiment of a nonwoven fabric constituting a leak-proof layer of an absorbent article according to the present invention.
{FIG. 4}
FIG. 4 is a partially enlarged cross-sectional drawing schematically showing another preferable embodiment of a nonwoven fabric constituting a leak-proof layer of an absorbent article according to the present invention.
{FIG. 5}
FIG. 5 is a drawing substitute photograph taking a cross section of the nonwoven fabric corresponding to FIG. 4.
{FIG. 6}
FIG. 6 is a drawing substitute photograph taken from a surface of a non-absorbent layer side of the nonwoven fabric shown in FIG. 5.

DESCRIPTION OF EMBODIMENTS

The present invention relates to an absorbent article including the leak-proof layer formed of a nonwoven fabric in which liquid leakage prevention is enhanced without adversely affecting softness.

Even in conventional nonwoven fabrics as described in JP-A-2016-65335 and JP-T-2011-529749 described above, or a meltblown nonwoven fabric in which interfiber distance is generally small, fine interfiber through-holes develop. When such a nonwoven fabric is applied as the leak-proof layer of an absorbent article, liquid leakage can be prevented up to some extent of liquid amount depending on through-hole size. However, there is a limit on this liquid amount.

A conceivable countermeasure is to close the through-holes to some extent by decreasing fiber diameter or increasing basis weight of the nonwoven fabric to thereby fill interfiber joints. However, since this method increases fiber amount, it may harden and adversely affect feel of the nonwoven fabric and therefore does not offer a viable solution to achieving a leak-proof layer in an absorbent article in contact with the skin.

In calender treatment used in a conventional method for producing nonwoven fabric, interfiber openings can be filled to some extent upon compressing the fibers. However, in fiber-free regions, it is difficult to completely fill the through-holes that become the cause of liquid leakage. If increase of thermocompression bonding by calender treatment is resorted to (compression bonding force is strengthened or compression bonding area is increased), hardening of the nonwoven fabric by flattening of fibers progresses and softness is lost accordingly.

On the other hand, the absorbent article according to the present invention includes a leak-proof layer formed of a nonwoven fabric in which liquid leakage prevention is enhanced without adversely affecting softness.

An absorbent article according to the present invention will be explained below based on a preferred embodiment thereof, referring to the drawings. The nonwoven fabric according to the present invention can be applied to various materials which absorbs and retains excreted liquid. For example, the nonwoven fabric can be used in a diaper, a sanitary napkin, a panty liner, an incontinence pad and a urine pad.

In the present invention, unless particularly stated otherwise, a side that is brought into contact with the human body is referred to as skin-facing surface side, skin-contacting surface side, or top surface side, and a side that is opposite to the above-mentioned side is referred to as non-skin-facing surface side, non-skin-contacting surface side, or back surface side. Especially, the skin-contacting surface side and the non-skin-contacting surface side of a leak-proof layer are referred to as an absorbent layer side and a non-absorbent layer side in several cases. The direction normal to the top surface or the back surface of the absorbent article is referred to as a thickness direction, and an amount thereof is referred to as thickness.

FIG. 1 shows a cross-section of an absorbent article 10 of the embodiment. The absorbent article 10 includes a liquid permeable top layer 1 arranged on the skin-contacting surface side, a leak-proof layer 2 arranged on the non-skin-contacting surface side, and a liquid-holding absorbent layer 3 disposed between the top layer 1 and the leak-proof layer 2. The absorbent layer 3 is a layer of a hydrophilic material arranged on the non-skin-contacting surface side of the top layer 1, and may contain not only an aggregate (absorbent core) of liquid absorbers such as pulp and an absorbent polymer but also a covering sheet (also referred to as a core wrap sheet) that covers the aggregate of liquid absorbers. Specific examples of the covering sheet include a liquid-permeable fiber sheet such as a thin paper (tissue paper) and a nonwoven fabric.

The leak-proof layer 2 is directly stacked on the absorbent layer 3. That is, the leak-proof layer 2 and the absorbent layer 3 are stacked without presence of other layers between both except for an agent (adhesive or the like) used for bonding. In the absorbent article 10 of the embodiment, the leak-proof layer 2 forms a layer closest to the non-skin-contacting surface side of the absorbent article 10. However, in the absorbent article according to the present invention, the leak-proof layer need not necessarily be the layer closest to the non-skin-contacting surface side. For example, when the absorbent article according to the present invention is a sanitary napkin or the like, an adhesive for fixing the article to clothing on the non-skin-contacting surface side of the leak-proof layer, a release paper for covering the adhesive until the adhesive is used, or the like may be arranged. The absorbent article 10 of the embodiment preferably includes no film on the non-skin-contacting surface side of the absorbent layer 3. The leak-proof layer 2 preferably includes a surface 21 on an absorbent layer side 2A in contact with the absorbent layer 3 and a surface 22 on a non-absorbent layer side 2B. The surface 22 on the non-absorbent layer side 2B of the leak-proof layer 2 preferably constitutes an outer surface of the absorbent article 10.

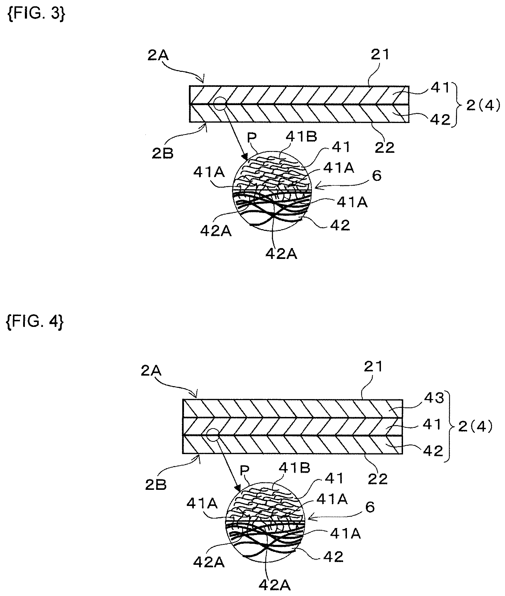

The leak-proof layer 2 includes a fiber layer 4 such as a nonwoven fabric or the like as a component. Specifically, as shown in FIG. 3, the leak-proof layer 2 has a fiber layer 4 including a meltblown layer 41 and a protective layer 42 of the meltblown layer 41. The protective layer 42 is preferably arranged at least on the non-absorbent layer side of the meltblown layer 41. The protective layer 42 is a layer which reinforces strength or the like of the meltblown layer 41. The leak-proof layer 2 preferably includes an embossed part (not shown) in which the meltblown layer 41 and the protective layer 42 are bonded, and a non-embossed part (not shown) other than the embossed part. The embossed part herein means a region in which constituent fibers of the fiber layer 4 are consolidated by heat embossing. In the embossed part formed in the fiber layer 4 by this consolidation, a thickness thereof is reduced, as compared with other sites of the fiber layer 4. The embossed part has a circular or rectangular shape, for example, and can be formed in a scatter form over a whole area of the fiber layer 4. Alternatively, a plurality of lines of linear or curved embossed parts can also be formed. The plurality of lines of the linear or curved embossed parts can also be formed so that the plurality of lines thereof may cross with each other.

The leak-proof layer 2 includes synthetic fibers as constituent fibers, and preferably has water-repellency. Thereby the leak-proof layer 2 includes a function of preventing the excreted liquid which is permeated from the top layer 1 and absorbed in the absorbent layer 3 from leaking to an outside of the absorbent article 10. Therefore the leak-proof layer 2 including the fiber layer 4 as a component preferably includes the following fiber structure in a region in which the leak-proof layer 2 overlaps with the absorbent layer 3.

Namely, the leak-proof layer 2 preferably includes, in a region in which the leak-proof layer 2 overlaps with the absorbent layer 3, a non-opening region in the non-embossed part. The non-opening region is a region which does not include the "through-hole in which, in a plane view, an interfiber space containing a region in which an area of a rectangle formed of 10 $\mu$m in a diagonal line length reaches 25 $\mu$m$^2$ or more passes therethrough in the thickness direction" (hereinafter, referred to as the through-hole). A size of the "region in which the area of the rectangle formed of 10 $\mu$m in the diagonal line length reaches 25 $\mu$m$^2$ or more" is a size at which water seepage from the leak-proof layer is worried about in the following state: namely, in the state in which the excreted liquid in the absorbent article arrives at the leak-proof layer from a top layer, and a seating pressure is applied to water (liquid such as the excreted liquid which is absorbed by the absorbent article). The through-hole is a hole which passes therethrough in the thickness direction at a predetermined size according to the definition described above, and the "non-opening region" which does not include the through-hole may be confirmed from either the surface 21 on the absorbent layer side 2A of the leak-proof layer 2, or the surface 22 on the non-absorbent layer side 2B thereof.

The "plane view" herein means to observe a sheet surface (the surface 21 on the absorbent layer side 2A, or the surface 22 on the non-absorbent layer side 2B) of the leak-proof layer 2 from above. The "hole which passes therethrough in the thickness direction" herein means a hole in which, "in the plane view, the interfiber space in which the area of the rectangle formed of 10 $\mu$m in the diagonal line length reaches 25 $\mu$m$^2$ or more" passes therethrough in the thickness direction of the leak-proof layer 2 while including the area.

Further, requirements for the diagonal line length and the area described above to be specified on the "through-hole" herein mean a seepage size upon application of the seating pressure to water in a relation with the excreted liquid to be absorbed in the absorbent article.

(Method for measuring through-hole)

Presence or absence of the through-hole can be measured by the method as follows.

That is, the sheet surface (the surface 21 on the absorbent layer side 2A or the surface 22 on the non-absorbent layer side 2B) of the leak-proof layer 2 is observed at an observation magnification of 3,000 times by using a scanning electron microscope (SEM) (manufactured by JEOL Co., Ltd., JCM-6000PLUS (trade name), for example, which is the same also in other places of this specification) to identify the interfiber space which passes therethrough without being blocked by the fibers when viewed from front to depth (the thickness direction of the leak-proof layer 2) of an observation screen (or image pick-up screen). Whether or not the rectangle (including a square) in which two straight lines in a 10 $\mu$m scale cross at a midpoint can be drawn relative to this interfiber space is examined. Then, when the area of the rectangle is 25 $\mu$m$^2$ or more, the through-hole is judged to be present.

The area of the rectangle is calculated based on the following Formula [1]. A smaller one of angles at the midpoint at which the straight lines cross in the rectangle mentioned above is taken as a crossing angle $\theta$ ($0° \leq \theta \leq 90°$) to measure the crossing angle $\theta$. The area of the rectangle in which the crossing angle $\theta$ is formed to be 30° or more with the diagonal line of 10 $\mu$m reaches 25 $\mu$m$^2$ or more.

$$\text{Area of rectangle} = (1/2) \times (\text{Diagonal line length: } 10 \text{ μm})^2 \times \sin \theta$$

$$[1]$$

When a leak-proof layer is taken out from a commercially available absorbent article for the measurement described above, a hotmelt adhering each inter-material is solidified by spraying a cold spray 10 cm away from the most exterior side for about 5 seconds, and then the leak-proof layer is carefully peeled off. The means of taking out the leak-proof layer is applied to other measuring methods in this specification.

The method for measuring a through-hole described above can be performed as shown in FIG. 2, for example. That is, when the leak-proof layer 2 is observed from the surface 21 on the absorbent layer side 2A, or the surface 22 on the non-absorbent layer side 2B, a space 49 which is surrounded by fibers 48, and is not blocked by other fibers in the thickness direction is identified. Subsequently, depending on whether or not a rectangle E in which two straight lines (drawn in the 10 $\mu$m scale and crossed at the midpoint, and the crossing angle is adjusted to be: $\theta$ = 30° or more and 90° or less) E1 and E1 are applied as the diagonal lines can be drawn, it is judged whether or not the through-hole defined above is present. When the rectangle E can be drawn, it is judged that an observation object leak-proof layer includes the through-hole. When the rectangle E cannot be drawn, it is judged that the observation object leak-proof layer does not include the through-hole.

A case where the leak-proof layer 2 does not include the through-hole defined above includes the aspect as described below, for example. That is, even if the interfiber space which satisfies the requirements for the diagonal line and the area described above is present on a front surface upon the plane view, when the fibers which block this interfiber space are present in depth thereof and the "hole" does not pass therethrough in the thickness direction with keeping the size described above, the leak-proof layer 2 does not include the through-hole described above. Also when a hole as small as the rectangle having the diagonal line of 10 $\mu$m in length cannot be drawn is present in the interfiber space which is visible on a front side upon observing the through-hole in the plane view, the leak-proof layer 2 does not include the through-hole described above, either. In the same manner, also when a small hole in which the area of the rectangle drawn so that two straight lines in the 10 $\mu$m scale may cross at the midpoint does not reach 25 $\mu$m$^2$ or more (namely, less than 25 $\mu$m$^2$) is present, the leak-proof layer 2 does not include the through-hole described above, either.

Specific examples of a method to enable the leak-proof layer 2 to have the non-opening region without the through-hole defined above include various methods, such as a method for increasing the number of fibers by increasing a basis weight.

Above all, from a viewpoint of keeping softness without excessively increasing a fiber amount to enhance liquid leakage prevention, the leak-proof layer 2 preferably includes a structure shown below.

That is, with regard to the leak-proof layer 2, in a laminated structure of the meltblown layer 41 and the protective layer 42 mentioned above, the leak-proof layer 2 includes a raised region 6 in which the number of fibers of the meltblown layer 41 for the fibers which penetrate into the protective layer 42 is 2.5 fibers/mm or more in the region which overlaps with the absorbent layer 3 (see a partially enlarged drawing in a circle shown by a symbol P in FIG. 3).

The "raised region 6" herein means a fiber structure inside the leak-proof layer 2, as described above. That is, inside the leak-proof layer 2, a part including a structure in which part of fibers (one end part of the fiber, for example) is protruded from a fiber aggregate 41B constituting the meltblown layer 41, and the protruded fibers (raised fibers) 41A penetrate into space between fibers 42A of the protective layer 42 is referred to as the "raised region 6". The protective layer 42 into which the fibers of the meltblown layer 41 penetrate is preferably arranged on the non-absorbent layer side 2B of the meltblown layer 41.

The laminated structure of the leak-proof layer 2 is not limited to an embodiment of a two-layer structure as shown in FIG. 3, and insofar as the laminated structure includes the protective layer 42 at least on the non-absorbent layer side 2B, the laminated structure may include a structure haing three or more layers. For example, as shown in FIG. 4, the laminated structure may also be a structure having another protective layer 43 on the absorbent layer side 2A in addition to the protective layer 42 on non-absorbent layer side 2B of the meltblown layer 41. Alternatively, although not shown, the meltblown layer 41 may be formed into two or more layers, or each of the protective layers 42 and 43 may be formed into two or more layers. However, from a viewpoint of keeping softness as the leak-proof layer 2, the leak-proof layer 2 has the "raised region 6 in which the number of fibers of the meltblown layer 41 for the fibers which penetrate into the protective layer 42 is 2.5 fibers/mm or more" described above with as few laminations as possible (see a partially enlarged drawing in a circle shown by a symbol P in FIGs. 3 and 4).

The raised region 6 mentioned above is present inside the leak-proof layer 2, thereby part of fibers of the meltblown layer 41 stands, and is arranged so that the fibers may bury the interfiber space of the leak-proof layer 2, even without excessively increasing the fiber amount of the leak-proof layer 2. As a result, the through-hole in the thickness direction is filled, and the leak-proof layer 2 has high liquid leakage prevention without adversely affecting softness.

From a viewpoint of liquid leakage prevention, adjustment of the number of fibers which penetrate thereinto in the raised region 6 of the leak-proof layer 2 to be 2.5 fibers/mm or more means the number of fibers which fill the through-hole mentioned above.

From a viewpoint of liquid leakage prevention, the number of fibers which penetrate from the meltblown layer 41 into the protective layer 42 in the raised region 6 is preferably 5 fibers/mm or more, and more preferably 10 fibers/mm or more. From a viewpoint of air permeability, the number of fibers which penetrate described above is preferably 100 fibers/mm or less, more preferably 50 fibers/mm or less, and further preferably 40 fibers/mm or less. Specifically, the number of fibers which penetrate from the meltblown layer 41 into the protective layer 42 in the raised region 6 is preferably 2.5 fibers/mm or more and 100 fibers/mm or less, more preferably 5 fibers/mm or more and 50 fibers/mm or less, and further preferably 10 fibers/mm or more and 40 fibers/mm or less.

(Method for measuring number of fibers of meltblown layer which penetrate into protective layer)

The number of fibers of the meltblown layer 41 which penetrate into the protective layer 42 can be measured by a method described below.

When a leak-proof layer is taken out from a commercially available absorbent article, the leak-proof layer 2 in the region which overlaps with the absorbent layer is taken out according to the method shown in (Method for measuring through-hole) mentioned above. The taken out leak-proof layer 2 is cut in the thickness direction to obtain a cross section. The leak-proof layer 2 is placed on an observation stage so that the cross section may face up to observe a cut surface at an observation magnification of 100 times by using the SEM.

With regard to a length (length in a plane direction in a leak-proof layer cross section) of a visual field, the visual field in one place when the leak-proof layer is observed by the SEM is adjusted to 1 mm to measure the numbers in ten places. The number of raised fibers 41A which penetrate from the meltblown layer 41 into the protective layer 42 on the non-absorbent layer side 2B is counted on each place. A total of the numbers counted in the ten places described above is taken as a numerator to calculate the number based on the following Formula [2].

(Number of fibers of meltblown layer for fibers which penetrate into

protective layer 42)

= (number of fibers of meltblown layer 41 for fibers which penetrate into

protective layer 42 in observation visual field) (fibers)

/ L (length of interface of protective layer 42 and meltblown layer 41 in

observation visual field (10 mm))

[2]

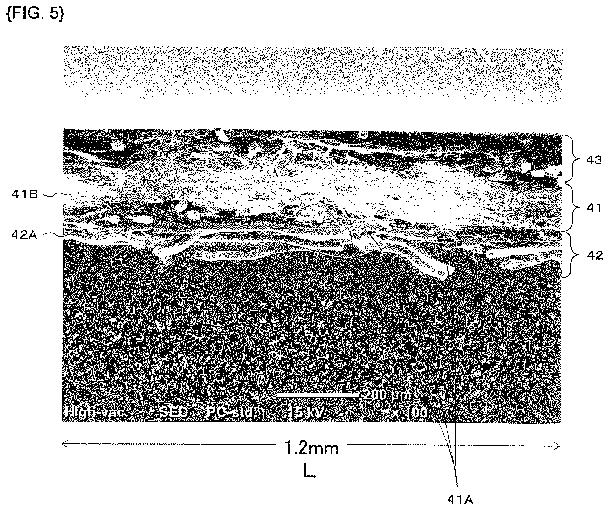

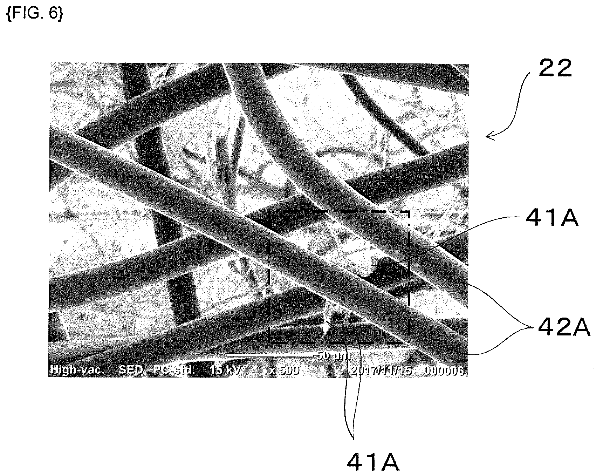

Specific examples of the cut surface in the thickness direction to be observed include a SEM image as shown in FIG. 5. In the cross section of the leak-proof layer 2 shown in FIG. 5, a three-layer structure of the protective layer (spunbond layer) 43 on the absorbent layer side 2A, the meltblown layer 41, and the protective layer (spunbond layer) 42 on the non-absorbent layer side 2B is formed. In the SEM image in FIG. 5, the raised fibers 41A protruded from the fiber aggregate 41B constituting the meltblown layer 41 penetrate into space between the fibers 42A of the protective layer 42 on the non-absorbent layer side 2B. The fibers of the meltblown layer 41 for the fibers which penetrate into the protective layer 42 are preferably formed as shown in a SEM image shown in FIG. 6, for example, when observed from the surface 22 on the non-absorbent layer side 2B. That is, as shown in a box region of a long dashed short dashed line in the SEM image of FIG. 6, the raised fibers 41A of the meltblown layer 41 are preferably formed into a state in which the raised fibers 41A are entangled with the fibers 42A of the protective layer 42, while the raised fibers 41A penetrate into space between the fibers 42A of the protective layer 42. Thereby penetration of the raised fibers 41A of the meltblown layer 41 into the protective layer 42 is further stably held.

In FIG. 5, as shown in FIG. 4, the protective layers 42 and 43 are arranged on both surfaces of the meltblown layer 41. In this case, as shown in FIG. 5, the raised region 6 is preferably arranged on the non-absorbent layer side 2B. This is because, from a viewpoint of liquid leakage prevention, a state of the raised fibers is held. More specifically, this is because a fiber structure of the raised region 6 is protected from a pressure of the liquid which migrates from the absorbent layer 3 to prevent the through-hole from being generated when in use of the absorbent article. The raised region 6 may be arranged on both the non-absorbent layer side 2B and the absorbent layer side 2A.

From a viewpoint of liquid leakage prevention, the raised region 6 is preferably arranged on a region as a whole in which the leak-proof layer 2 overlaps with the absorbent layer 3. The raised region 6 is more preferably arranged from a region which overlaps with the absorbent layer 3 to a plane region which does not overlap with the absorbent layer 3 beyond this region.

In the leak-proof layer 2, the fibers of the meltblown layer 41 preferably are not exposed on the top surface (top surface of the surface 22) on the non-absorbent layer side 2B. As an aspect in which the fibers of the meltblown layer 41 are not exposed thereon, a thickness of the protective layer 42 on the non-absorbent layer side 2B of the meltblown layer 41 can be adjusted to a predetermined level, or the fibers can also be immobilized by using a hot-melt adhesive or the like, for example.

Thus, the fibers of the meltblown layer 41 are not exposed on the top surface (top surface of the surface 22) on the non-absorbent layer side 2B, and thereby the raised fibers of the meltblown layer 41 are protected, the fiber structure of the raised region 6 is held, and the liquid becomes difficult to seep out from the leak-proof layer 2.

(Method for confirming whether or not fibers of meltblown layer are exposed on top surface of leak-proof layer)

Presence or absence of the exposure described above can be confirmed by observing a top surface of an observation object at an observation magnification of 100 times by using the SEM.

When the fibers of the meltblown layer 41 are reflected on a topmost surface of the observation screen, the fibers of the meltblown layer are judged to be exposed on the top surface. If not, for example, when only the fibers of the protective layer are reflected on the topmost surface of the observation screen, the fibers are judged not to be exposed thereon.

The "meltblown layer" herein means a fiber layer (layer of a nonwoven fabric of a type directly joined with spinning) formed by spinning a heated and melted thermoplastic resin, and allowing the resulting fibers to accumulate on a conveyor, thereby being processed into the nonwoven fabric according to a so-called meltblown method. In the meltblown method, the melted resin output from nozzles of a spinneret is blown off by a jet flow of high-temperature gas to form ultra-micronized floc fibers in a tearing off manner. Therefore, the obtained meltblown layer is a layer of the nonwoven fabric formed of ultrafine fibers having a fiber diameter of 10 μm or less. Constituent fibers are formed of filament fibers having a fiber length of 30 μm or more. Interfiber distance is also small. Such the meltblown layer 41 is superior in feelings and barrier properties. On the other hand, reinforcement by the protective layer 42 is required on strength or wear resistance.

From a viewpoint of excellent liquid leakage prevention, an average fiber diameter (R1) of fibers of the meltblown layer 41 is 2.5 μm or less, preferably 1.5 μm or less, and further preferably 1 μm or less. The average fiber diameter (R1) is practically 0.1 μm or more. Specifically, the average fiber diameter (R1) of fibers of the meltblown layer 41 is preferably 0.1 μm or more and 2.5 μm or less, more preferably 0.1 μm or more and 1.5 μm or less, and further preferably 0.1 μm or more and 1 μm or less. In the laminated structure of the meltblown layer 41 and the protective layer 42 of the leak-proof layer 2, the average fiber diameter (R1) of fibers of the meltblown layer 41 is suppressed within the range described above, and thereby the interfiber space can be further narrowed to more definitely prevent the leak-proof layer 2 from including the through-hole mentioned above.

The protective layer 42 is a fiber layer which reinforces the strength or the like of the meltblown layer 41, and is preferably formed by using fibers having a larger fiber diameter than a fiber diameter of the meltblown layer 41. As the protective layer 42, insofar as the layer may reinforce the strength or the like of the meltblown layer 41, various fiber layers can be used. Among these, from a viewpoint of cost, the protective layer 42 is preferably formed of a spunbond layer.

The "spunbond layer" herein means a layer of a nonwoven fabric which is produced by a so-called spunbond method. In the same manner as in the meltblown layer, the spunbond layer is a fiber layer formed by spinning the heated and melted thermoplastic resin and allowing the resulting fibers to accumulate on a conveyor. However, in the spunbond method, the fibers are formed while cooling and drawing a melted resin output from nozzles from a spinneret. The obtained spunbond layer has larger fiber diameter than a fiber diameter of the meltblown layer, and is a fiber layer formed of fibers having long fiber length (filament). It is preferable that the spunbond layer is laminated on the meltblown layer, and then heat embossing is applied thereto, and the resulting product is processed into the nonwoven fabric in which both are integrated.

From a viewpoint of more definitely preventing the leak-proof layer 2 from including the through-hole mentioned above, from a viewpoint of further increasing the number of fibers of the meltblown layer 41 for the fibers which penetrate into the protective layer 42 in the raised region 6, and from a viewpoint of further enhancing effectiveness of a protective function of the protective layer 42 to the meltblown layer 41, the average fiber diameter (R1) of fibers of the meltblown layer 41 is preferably smaller than an average fiber diameter (R2) of fibers of the protective layer 42. From these viewpoints, a ratio (R1/R2) of the average fiber diameter (R1) of fibers of the meltblown layer 41 to the average fiber diameter (R2) of fibers of the protective layer 42 is preferably 1/8 or less, more preferably 1/20 or less, and further preferably 1/25 or less. From a viewpoint of air permeablity, the ratio (R1/R2) is preferably 1/100 or more, more preferably 1/50 or more, and further preferably 1/40 or more. Specifically, the ratio (R1/R2) is preferably 1/100 or more and 1/8 or less, more preferably 1/50 or more and 1/20 or less, and further preferably 1/40 or more and 1/25 or less.

(Method for measuring average fiber diameter)

An art is referred to, in which five small-piece samples are collected from a meltblown layer of a leak-proof layer in a random manner, a photograph in which an observation magnification is increased, for example, to 1,000 to 10,000 times is taken so that 20 to 60 fibers may be taken in a visual field by using the SEM, a fiber diameter is measured on all the fibers within the visual field so that the fiber may be counted once for each, and the measured average value is rounded off to a first decimal place to calculate and determine an average fiber diameter.

In the meltblown layer 41, from a viewpoint of holding softness while filling the through-hole of the leak-proof layer 2, a filling rate is adjusted to preferably 25% or less, more preferably 20% or less, further preferably 10% or less, and particularly preferably 5% or less. The "filling rate" herein means a proportion of fibers per space. The filling rate of the meltblown layer 41 is suppressed within the range described above. Thereby as texture of the meltblown layer 41 alone, the layer can include flexibility which may produce a gentle curved surface or pleat as in cloth (high drape property) to suppress hardness as in paper or a film (crisply feeling: hardness which needs a certain degree of force upon folding or the like, and is easily bent, and not curved upon twisting, for example: dry chafing sound which is generated when touched, or when twisted or the like).

From a viewpoint of leakage prevention, the filling rate of the meltblown layer 41 is preferably 2% or more, more preferably 2.5% or more, and further preferably 3% or more. Specifically, the filling rate of the meltblown layer 41 is preferably 2% or more and 25% or less, more preferably 2% or more and 20% or less, further preferably 2.5% or more and 10% or less, and particularly preferably 3% or more and 5% or less.

(Method for measuring filling rate of meltblown layer)

The filling rate of the meltblown layer can be measured by the method described below.

Mass of a measurement object leak-proof layer is measured. A cross section in a thickness direction of the leak-proof layer is observed by using the SEM described above, and the thickness of the meltblown layer is measured. The meltblown layer is taken out based on (Method for measuring basis weight of meltblown layer) described later. The basis weight is calculated, and the filling rate is calculated by the expression: { basis weight of meltblown layer / (thickness of meltblown layer $\times$ density of resin) } $\times$ 100.

The "resin density" described above can be measured by the method described below.

That is, the meltblown layer taken out therefrom is pressed at 180°C by LABO PRESS (manufactured by Toyo Seiki Seisaku-Sho, Ltd., model P2-30), and for 1 minute by a two-step press (low pressure: 5 kg/cm$^3$, high pressure: 150 kg/cm$^3$), and then for 1 minute by a cooling press to prepare a film. Then, the film is cut into 10 $\times$ 10 cm from a place in which air is not entrained, mass is measured, and then the resulting value is divided by a volume to calculate the resin density.

From a viewpoint of securing softness, the basis weight of the meltblown layer 41 is preferably 20 g/m$^2$ or less, more preferably 15 g/m$^2$ or less, and further preferably 10 g/m$^2$ or less. From a viewpoint of securing sheet strength, the basis weight of the meltblown layer 41 is preferably 3 g/m$^2$ or more, more preferably 4 g/m$^2$ or more, and further preferably 5 g/m$^2$ or more. Specifically, the basis weight of the meltblown layer 41 is preferably 3 g/m$^2$ or more and 20 g/m$^2$ or less, more preferably 4 g/m$^2$ or more and 15 g/m$^2$ or less, and further preferably 5 g/m$^2$ or more and 10 g/m$^2$ or less.

(Method for measuring basis weight of meltblown layer)

The basis weight of the meltblown layer can be measured as described below.

Mass of a measurement object leak-proof layer is measured in a dry state. A cross section in a thickness direction of the leak-proof layer is observed by using the SEM described above, and the thickness of each of the meltblown layer and the protective layer and a thickness ratio thereof are measured. In order to peel off the meltblown layer from the leak-proof layer, the following means are used. When the meltblown layer and the protective layer are bonded with the hot-melt or the like, the meltblown layer is carefully peeled off using a cold spray. Mass of the meltblown layer is measured, and the resulting value is divided by a sheet area of the leak-proof layer to calculate the basis weight of the meltblown layer. When the meltblown layer and the protective layer are thermally bonded by embossing or the like, an embossed part is removed, and then the meltblown layer is carefully peeled off by hand, tweezers, or the like. Then, the mass of the meltblown layer is measured, and the resulting value is divided by a value obtained by subtracting an area of the embossed part from the sheet area of the leak-proof layer to calculate the basis weight of the meltblown layer.

A preferable method for producing a nonwoven fabric serving as the leak-proof layer 2 of the embodiment will be described. A case where the protective layer 42 is applied as a spunbond layer will be described herein.

In the meltblown layer 41, ultrafine fibers are accumulated on a belt conveyor by a meltblown method to continuously convey a meltblown layer raw material in a machine direction. On the occasion, the fiber diameter, the filling rate, and the basis weight mentioned above can be satisfied by setting various conditions such as a diameter of the nozzle of the spinneret, an air speed and a temperature of the jet flow of high-temperature gas. Subsequently, as the protective layer 42, a spunbond layer raw material is laminated on a surface of the meltblown layer raw material described above by the spunbond method. On the occasion, raising treatment is preferably applied to the surface of the meltblown layer raw material in an upstream of a front in which the spunbond layer raw material is laminated thereon. Thereby the spunbond layer raw material is laminated on a raising-treated surface of the meltblown layer raw material, and the raised region 6 mentioned above is formed.

The raising treatment can be conducted by various methods ordinarily used for a method for producing a nonwoven fabric. For example, the raising treatment is conducted by rotating a convex roll having a plurality of convex parts on a peripheral surface while the convex roll is brought into contact with the surface of the meltblown layer raw material. Thereby the raised fibers can be formed.

After the raising treatment, on the raising-treated surface of the meltblown layer raw material, the fibers that are output, cooled and drawn by the spunbond method and serving as the spunbond layer raw material are accumulated. Thereby the raised fibers of the meltblown layer raw material penetrate into space between the fibers of the spunbond layer raw material, and the raised region 6 in the leak-proof layer 2 of the embodiment is formed. Thereby the nonwoven fabric serving as the leak-proof layer 2 of the embodiment can be produced.

In this producing method, a structure of the "number of fibers of the meltblown layer for the fibers which penetrate into the protective layer is 2.5 fibers/mm or more" in the raised region 6 can be formed by protrusion of part of fibers (one end part of the fiber, for example) from the fiber aggregate 41B constituting the meltblown layer 41 and penetration of the protruded fibers (raised fibers) 41A into space between the fibers 42A of the protective layer 42 on the non-absorbent layer side 2B. In the method for producing the nonwoven fabric serving as the leak-proof layer 2 of the embodiment, a desired average fiber diameter, filling rate, basis weight or the like can be obtained by appropriately setting various production conditions.

In the producing method described above, a protective layer 43 formed of the spunbond layer can be further arranged on a surface serving as the absorbent layer side 2A of the meltblown layer raw material. In this case, a step for producing the spunbond layer raw material in the same manner described above may further be provided in an upstream of the step for producing the meltblown layer raw material. In this case, the meltblown layer raw material is formed on the spunbond layer raw material produced in an upstream.

As the method for producing an absorbent article according to the present invention, it is preferable that the nonwoven fabric obtained by the producing method described above is directly stacked on the absorbent layer as the leak-proof layer. The top layer is stacked on the absorbent layer with the leak-proof layer (nonwoven fabric) which are directly stacked on the skin-contacting surface side of the absorbent layer, and the resulting product is integrated to produce the absorbent article according to the present invention. In an occasion, the method may include a step of assembling other members, when necessary.

Constituent fibers of the meltblown layer 41 of the leak-proof layer 2, and constituent fibers in the case where the protective layers 42 and 43 are formed of spunbond layers, are formed of a thermoplastic resin. Specific examples of the thermoplastic resins include one or more selected from a polyolefin-based resin, a polyester-based resin, a polyamide-based resin, an acrylonitrile-based resin, a vinyl-based resin and a vinylidene-based resin. Specific examples of the polyolefin-based resin include one or more selected from polyethylene, polypropylene and polybutene. Specific examples of the polyester-based resin include one or more selected from polyethylene terephthalate and polybutylene terephthalate. Specific examples of the polyamide-based resin include one or more selected from nylon and the like. Specific examples of the vinyl-based resin include polyvinyl chloride and the like. Specific examples of the vinylidene-based resin include polyvinylidene chloride. A modified product or a mixture of various resins described above can also be used.

EXAMPLES

Hereinafter, the present invention will be described more in detail with reference to Examples, but the present invention is not limited thereto.

(Example 1)

A spunbond layer raw material is formed having an average fiber diameter of 20 $\mu$m and a basis weight of 9 g/m$^2$, using a polyolefin resin by a spunbond method.

A meltblown layer raw material is formed having a basis weight of 10 g/m$^2$ thereon, using a polyolefin resin by a meltblown method (spinning temperature: 270°C). Raising treatment was conducted onto a whole surface of the meltblown layer raw material. Specifically, the meltblown layer raw material was chafed once at 1.2 m/min in a machine direction of the meltblown layer raw material, while applying a pressure of 165 Pa thereto, by Sheet Paper, manufactured by TRUSCO NAKAYAMA CORPORATION, catalog number: GBS-600-5P. An average fiber diameter, a thickness, and a filling rate of the obtained meltblown layer were measured based on (Method for measuring average fiber diameter), (Method for measuring filling rate of meltblown layer), and (Method for measuring basis weight of meltblown layer) described above.

Subsequently, on a raising-treated surface of the meltblown layer raw material, the spunbond layer raw material having the average fiber diameter of 20 $\mu$m and the basis weight of 9 g/m$^2$ was formed, using the polyolefin resin by the spunbond method.

Then, heat embossing was applied to a laminated spunbond-meltblown-spunbond (SMS) fiber layer, and the resulting product was taken as an SMS nonwoven fabric.

Thereby a leak-proof layer sample in Example 1 was prepared, in which spunbond layers arranged on both surfaces of a meltblown layer 41 were applied as protective layers 42 and 43.

In the obtained leak-proof layer sample, on a surface with the spunbond layer on a raising-treated side of the meltblown layer 41, the number of fibers of the meltblown layer 41 for the fibers which penetrated into the protective layer from a raising-treated surface of the meltblown layer 41 was measured based on (Method for measuring number of fibers which penetrate thereinto) mentioned above, and was shown as in Table 1 (hereinafter, the same in Examples 2 and 3).

Similarly, in the obtained leak-proof layer sample, on the surface with the spunbond layer on the raising-treated side of the meltblown layer 41, whether or not the fibers of the meltblown layer were exposed on a top surface of the spunbond layer was confirmed based on (Method for confirming whether or not fibers of meltblown layer are exposed on top surface of leak-proof layer) mentioned above, and was shown as in Table 1 (hereinafter, the same in Examples 2 and 3).

(Example 2)

A leak-proof layer sample in Example 2 was prepared in the same manner as in Example 1 except that a meltblown layer 41 was applied as shown in Table 1. The fiber diameter described above was realized by changing a spinning temperature of the meltblown layer in Example 1 to a low temperature (255°C).

(Example 3)

A leak-proof layer sample in Example 3 was prepared in the same manner as in Example 1 except that a meltblown layer 41 was applied as shown in Table 1. The fiber diameter described above was realized by changing a spinning temperature of the meltblown layer in Example 1 to 265°C.

(Comparative Example 1)

A leak-proof layer sample in Comparative Example 1 was prepared in the same manner as in Example 1 except that a meltblown layer 41 was applied as described in Table 1 without conducting raising treatment thereto. Formation of fibers which slightly penetrated thereinto, irrespective of no raising treatment, was caused by a pressure applied thereto upon lamination.

(Comparative Example 2)

A leak-proof layer sample in Comparative Example 2 was prepared in the same manner as in Example 2 except that a meltblown layer 41 was applied as described in Table 1 without conducting raising treatment thereto.

(Comparative Example 3)

A leak-proof layer sample in Comparative Example 3 was prepared in the same manner as in Example 1 except that a meltblown layer 41 was applied as shown in Table 1. The fiber diameter was realized by changing a spinning temperature of the meltblown layer in Example 1 to a low temperature (250°C).

(Comparative Example 4)

A leak-proof layer sample in Comparative Example 4 was prepared in the same manner as in Example 3 except that a meltblown layer 41 was applied as described in Table 1 by enhancing a filling rate by calender treatment without conducting raising treatment thereto.

(Comparative Example 5)

A spunbond layer raw material and a meltblown layer raw material which were the same as in Example 2 were prepared one for each, and in a state in which both raw materials were overlapped, a water flow was jetted twice at water pressures different from each other from a meltblown layer raw material side. First jetting was performed at a low pressure (2.5 MPa) using a nozzle having a diameter of 0.12 mm to entangle constituent fibers of the spunbond layer raw material and the meltblown layer raw material, and simultaneously both layers were wetted with water and well fitted. Second jetting was performed at a high pressure (5 MPa) using a nozzle having a diameter of 0.1 mm to further entangle the constituent fibers of the spunbond layer raw material and the meltblown layer raw material to obtain an SM nonwoven fabric formed of two layers of a spunbond-meltblown (SM).

Thereby a leak-proof layer sample in Comparative Example 5 was prepared, in which a spunbond layer arranged on one surface of a meltblown layer 41 was applied as a protective layer 42. A thickness and a filling rate of the meltblown layer were measured based on (Method for measuring filling rate of meltblown layer) and (Method for measuring basis weight of meltblown layer) mentioned above.

In the leak-proof layer sample in Comparative Example 5, the number of fibers of the meltblown layer 41 for the fibers which penetrated into the protective layer 42 from a surface of the meltblown layer 41 was measured based on (Method for measuring number of fibers which penetrate thereinto) mentioned above, and was shown in Table 1.

(Comparative Example 6)

A leak-proof layer sample in Comparative Example 6 was prepared in the same manner as in Comparative Example 5 except that a pressure of a water flow to be jetted was adjusted to 1.75 MPa for first jetting, and to 3.5 MPa for second jetting.

(Comparative Example 7)

A leak-proof layer sample in Comparative Example 7 was prepared in the same manner as in Comparative Example 5 except that a pressure of a water flow to be jetted was adjusted to 1 MPa for first jetting, and to 2 MPa for second jetting.

Confirmations and tests described below were conducted on each leak-proof layer samples in Examples and Comparative Examples described above. The results are shown in Table 1.

(1) Liquid leakage prevention test:

On a filter paper (manufactured by Advantech Toyo Co., Ltd., No. 2, diameter: 70 mm), a specimen prepared by cutting the leak-proof layer sample into 8 cm × 8 cm was placed. On the occasion, the leak-proof layer sample was placed thereon with a surface on a non-absorbent layer side 2B (surface with a raised region) directed toward the filter paper. On the leak-proof layer sample, a specimen prepared by cutting a dry pulp sheet (manufactured by Lion Corporation, Lead Healthy Cooking Paper Double (trade name), basis weight: 40 g/m$^2$) into 8 cm × 8 cm was placed.

Subsequently, from above the dry pulp sheet, 1.0 g of deionized water into which 0.005 mass% of Blue No. 1 was incorporated was injected thereinto using a dropping pipet. After injection, an acrylic plate having a diameter of 60 mm was overlapped thereon, and was pressurized for 5 seconds using a 2 kg weight from above.

After pressurization for 5 seconds, presence or absence of wetting in the filter paper (presence or absence of seepage) was confirmed by a method for visually confirming coloring to the filter paper. An amount of deionized water seeped out to the filter paper was confirmed by subtracting a weight before testing from a weight after testing as measured by an electronic balance.

1.0 g of deionized water injected thereinto was used by assuming a state in which a whole leak-proof layer is wet with a liquid migrated from an absorbent layer to the leak-proof layer in an absorbent article. A load of 2 kg weight was applied by assuming a state in which a seating pressure was applied by wearing the absorbent article.

(2) Softness test:

Sensory evaluation of softness was performed by asking five panels (persons engaged in research in an absorbent article field) to rub surfaces (both surfaces) of each nonwoven fabric sample by hand. After asking each panel to score the evaluation based on the evaluation criteria described below, an average value of evaluation scores of five persons was taken as a score of the sensory evaluation of softness of each sample.

A hot-melt was dissolved by using an organic solvent (butyl acetate) for a disposable diaper (manufactured by Kao Corporation, Merries (registered trademark), Merries Pants, M size, 2017) to remove the most exterior (non-skin surface side) nonwoven fabric and a film being the leak-proof layer, and the resulting specimen was dried for one day in a draft, and then was used. Softness of each leak-proof layer sample when softness of this most exterior (non-skin surface side) nonwoven fabric was rated as 5 points, and softness of the film being the leak-proof layer was rated as 1 point was quantified in five scales.

Table 1

| | Example 1 | Example 2 | Example 3 | Comparative Example 1 | Comparative Example 2 |
|---|---|---|---|---|---|
| Nonwoven fabric structure | SMS | SMS | SMS | SMS | SMS |
| Means being processed into nonwoven fabric | Heat-embossing | Heat-embossing | Heat-embossing | Heat-emboss-ing | Heat-emboss-ing |

(continued)

| | | Example 1 | Example 2 | Example 3 | Comparative Example 1 | Comparative Example 2 |
|---|---|---|---|---|---|---|
| Meltblown layer | Average fiber diameter (μm) | 0.8 | 2.4 | 1.1 | 0.8 | 2.4 |
| | Basis weight (g/m²) | 10 | 10 | 10 | 10 | 10 |
| | Thickness (μm) | 352 | 254 | 244 | 317 | 244 |
| | Filling rate (%) | 3.1 | 4.3 | 4.5 | 3.5 | 4.5 |
| Raising treatment | | Whole surface | Whole surface | Whole surface | Untreated | Untreated |
| Fibers of meltblown layer which penetrate into protective layer (fibers/mm) | | 30.0 | 14.2 | 3.3 | 0.8 | 0.0 |
| Presence or absence of through-hole | | Absence | Absence | Absence | Presence | Presence |
| Presence or absence of exposure of fibers of meltblown layer | | Absence | Absence | Absence | Absence | Absence |
| Liquid leakage prevention (mg) | | 0 | 0 | 0 | 4 | 14 |
| Softness | | 3.4 | 4.2 | 3.6 | 3.6 | 4.2 |

Table 1 (continued)

| | | Comparative Example 3 | Comparative Example 4 | Comparative Example 5 | Comparative Example 6 | Comparative Example 7 |
|---|---|---|---|---|---|---|
| Nonwoven fabric structure | | SMS | SMS | SM | SM | SM |
| Means being processed into nonwoven fabric | | Heat-embossing | Heat-embossing | Hydroentangling | Hydroentangling | Hydroentangling |
| Meltblown layer | Average fiber diameter (μm) | 3.5 | 1.1 | 2.4 | 2.4 | 2.4 |
| | Basis weight (g/m²) | 10 | 10 | 10 | 10 | 10 |
| | Thickness (μm) | 234 | 43 | 145 | 168 | 182 |
| | Filling rate (%) | 4.7 | 25.8 | 7.6 | 6.5 | 6.0 |
| Raising treatment | | Whole surface | Untreated | Untreated | Untreated | Untreated |
| Fibers of meltblown layer which penetrate into protective layer (fibers/mm) | | 24.2 | 0.0 | 23.3 | 19.2 | 7.5 |
| Presence or absence of through-hole | | Presence | Presence | Presence | Presence | Presence |

(continued)

|  | Comparative Example 3 | Comparative Example 4 | Comparative Example 5 | Comparative Example 6 | Comparative Example 7 |
|---|---|---|---|---|---|
| Presence or absence of exposure of fibers of meltblown layer | Absence | Absence | Presence | Presence | Presence |
| Liquid leakage prevention (mg) | 27 | 24 | 193 | 121 | 72 |
| Softness | 4.4 | 2.0 | 3.4 | 3.4 | 3.2 |

As shown in Table 1 described above, the leak-proof layer samples in Examples 1 to 3 in which the number of fibers of the meltblown layer for the fibers which penetrated into the protective layer was 2.5 fibers/mm or more and the layers had no through-holes were superior in liquid leakage prevention to the leak-proof layer samples in Comparative Example 1, 2 and 4 in which the number of fibers of the meltblown layer for the fibers which penetrated into the protective layer was less than 2.5 fibers/mm and the layers had the through-holes. In the same manner, the leak-proof layer samples in Examples 1 to 3 in which the layers had no through-holes were superior in liquid leakage prevention, even in contrast with the leak-proof layer samples in Comparative Examples 3 and 5 to 7 in which the layers had the through-holes, even if the number of fibers of the meltblown layer for the fibers which penetrated into the protective layer was 2.5 fibers/mm.

In particular, the leak-proof layer samples in Examples 1 to 3 had significantly higher liquid leakage prevention in contrast with the leak-proof layer samples in Comparative Examples 5 to 7. This is because, while the raised region was formed by a hydroentangling method, thereby causing movement of fibers of the meltblown layer by a pressure of the water flow to develop the through-holes of 25 $\mu m^2$ or more in Comparative Examples 5 to 7, the raised region was formed by raising treatment, and further the movement of fibers of the meltblown layer was able to be suppressed by heat embossing in Examples 1 to 3. Further, this is also because exposure of fibers of the meltblown layer to the top surface on the non-absorbent layer side of the leak-proof layer was able to be prevented in the leak-proof layer samples in Examples 1 to 3.

In addition thereto, it was found that softer texture can also be realized by suppressing the filling rate of the meltblown layer in Examples 1 to 3.

DESCRIPTION OF SYMBOLS

1 Top layer
2 Leak-proof layer
3 Absorbent layer
4 Fiber layer
6 Raised region
2A Absorbent layer side of leak-proof layer
2B Non-absorbent layer side of leak-proof layer
21 Surface of absorbent layer side of leak-proof layer
22 Surface of non-absorbent layer side of leak-proof layer
41 Meltblown layer
41A Raised fiber of meltblown layer
42, 43 Protective layer
42A Fiber of protective layer
48 Fiber
49 Space
10 Absorbent article

**Claims**

1. An absorbent article, comprising a top layer (1), a leak-proof layer (2), and an absorbent layer (3) disposed between the top layer (1) and the leak-proof layer (2);

   wherein the leak-proof layer (2) is directly stacked on the absorbent layer (3),

and the leak-proof layer (2) comprises a meltblown layer (41) and a protective layer (42) of the meltblown layer (41);

wherein the protective layer (42) is arranged on a non-absorbent layer side of the meltblown layer (41);

wherein the leak-proof layer (2) comprises a raised region (6) in which the number of fibers of the meltblown layer (41) for the fibers which penetrate into the protective layer (42) is 2.5 fibers/mm or more, the number of fibers being measured with a method as described in the description;

wherein an average fiber diameter of fibers of the meltblown layer (41) is 2.5 $\mu$m or less; and

wherein the raised region (6) is arranged on a region in which the leak-proof layer (2) overlaps with the absorbent layer (3).

2. The absorbent article according to Claim 1

wherein the leak-proof layer (2) comprises an embossed part in which the meltblown layer (41) and the protective layer (42) are bonded, and a non-embossed part other than the embossed part;

wherein the leak-proof layer (2) comprises a non-opening region in the non-embossed part;

wherein the non-opening region is a region which does not comprise a through-hole in which, in a plane view of the leak-proof layer, an interfiber space containing a region in which an area of a rectangle formed of 10 $\mu$m in a diagonal line length reaches 25 $\mu$m$^2$ or more passes therethrough in a thickness direction, the through-hole being measured with a method as described in the description; and

wherein the non-opening region is arranged on a region in which the leak-proof layer (2) overlaps with the absorbent layer (3).

3. The absorbent article according to Claim 1 or 2, wherein the raised region (6) or the non-opening region is arranged on a region as a whole in which the leak-proof layer (2) overlaps with the absorbent layer (3).

4. The absorbent article according to any one of Claims 1 to 3, wherein the leak-proof layer (2) comprises another protective layer (43) on the absorbent layer side (3) in addition to the protective layer (42) on the non-absorbent layer side of the meltblown layer (41).

5. The absorbent article according to Claim 4, wherein the raised region (6) is arranged on the absorbent layer side in addition to the non-absorbent layer side.

6. The absorbent article according to any one of Claims 1 to 5, wherein the number of fibers which penetrate from the meltblown layer (41) into the protective layer (42) of the non-absorbent layer side is preferably 2.5 fibers/mm or more and 100 fibers/mm or less, more preferably 5 fibers/mm or more and 50 fibers/mm or less, and further preferably 10 fibers/mm or more and 40 fibers/mm or less.

7. The absorbent article according to any one of Claims 1 to 6, wherein the raised region (6) or the non-opening region is arranged from the region which overlaps with the absorbent layer (3) to a plane region which does not overlap with the absorbent layer (3) beyond this region.

8. The absorbent article according to any one of Claims 1 to 7, wherein the average fiber diameter of fibers of the meltblown layer (41) is preferably 0.1 $\mu$m or more and 2.5 $\mu$m or less, more preferably 0.1 $\mu$m or more and 1.5 $\mu$m or less, and further preferably 0.1 $\mu$m or more and 1 $\mu$m or less.

9. The absorbent article according to any one of Claims 1 to 8, wherein a ratio of the average fiber diameter of fibers of the meltblown layer (41) to an average fiber diameter of fibers of the protective layer (42) is preferably 1/100 or more and 1/8 or less, more preferably 1/50 or more and 1/20 or less, and further preferably 1/40 or more and 1/25 or less.

10. The absorbent article according to any one of Claims 1 to 9, wherein a basis weight of the meltblown layer (41) is preferably 3 g/m$^2$ or more and 20 g/m$^2$ or less, more preferably 4 g/m$^2$ or more and 15 g/m$^2$ or less, and further preferably 5 g/m$^2$ or more and 10 g/m$^2$ or less.

11. A method for producing a nonwoven fabric for use in an absorbent article according to claim 1, wherein ultrafine fibers are accumulated on a belt conveyor by a meltblown method to continuously convey a meltblown layer (41) raw material in a machine direction;

raising treatment is applied to a surface of the meltblown layer raw material; and

a spunbond layer raw material is laminated on a raising-treated surface of the meltblown layer raw material by a spunbond method.

**Patentansprüche**

1. Absorbierender Artikel, der eine Deckschicht (1), eine auslaufsichere Schicht (2) und eine zwischen der Deckschicht (1) und der auslaufsicheren Schicht (2) angeordnete absorbierende Schicht (3) aufweist,

   wobei die auslaufsichere Schicht (2) direkt auf die absorbierende Schicht (3) gestapelt ist und die auslaufsichere Schicht (2) eine schmelzgeblasene Schicht (41) und eine Schutzschicht (42) der schmelzgeblasenen Schicht (41) aufweist,
   wobei die Schutzschicht (42) auf einer nichtabsorbierenden Seite der schmelzgeblasenen Schicht (41) angeordnet ist,
   wobei die auslaufsichere Schicht (2) einen erhöhten Bereich (6) aufweist, in dem die Anzahl der Fasern der schmelzgeblasenen Schicht (41) für die Fasern, die in die Schutzschicht (42) eindringen, mindestens 2,5 Fasern/mm beträgt, wobei die Anzahl der Fasern mit einem Verfahren gemessen wird, das in der Beschreibung beschrieben ist,
   wobei ein durchschnittlicher Faserdurchmesser der Fasern der schmelzgeblasenen Schicht (41) höchstens 2,5 μm beträgt und
   wobei der erhöhte Bereich (6) in einem Bereich angeordnet ist, in dem die auslaufsichere Schicht (2) mit der absorbierenden Schicht (3) überlappt.

2. Absorbierender Artikel nach Anspruch 1, wobei die auslaufsichere Schicht (2) einen geprägten Teil aufweist, in dem die schmelzgeblasene Schicht (41) und die Schutzschicht (42) verklebt sind, und einen nicht-geprägten Teil, der sich von dem geprägten Teil unterscheidet,

   wobei die auslaufsichere Schicht (2) einen nicht-öffnenden Bereich in dem nicht-geprägten Teil aufweist,
   wobei der nicht-öffnende Bereich ein Bereich ist, der keine Durchgangsöffnung aufweist, in dem in einer zweidimensionalen Ansicht der auslaufsicheren Schicht ein Zwischenfaserraum mit einem Bereich, in dem eine Fläche eines Rechtecks mit einer Diagonallänge von 10 μm mindestens 25 μm² erreicht, in einer Dickenrichtung hindurchgeht, wobei das Durchgangsloch mit einem Verfahren, das in der Beschreibung beschrieben ist, gemessen wird, und
   wobei der nicht-öffnende Bereich in einem Bereich angeordnet ist, in dem die auslaufsichere Schicht (2) mit der absorbierenden Schicht (3) überlappt.

3. Absorbierender Artikel nach Anspruch 1 oder 2, wobei der erhöhte Bereich (6) oder der nicht-öffnende Bereich insgesamt in einem Bereich angeordnet ist, in dem die auslaufsichere Schicht (2) mit der absorbierenden Schicht (3) überlappt.

4. Absorbierender Artikel nach einem der Ansprüche 1 bis 3, wobei die auslaufsichere Schicht (2) zusätzlich zu der Schutzschicht (42) auf der Seite der nicht-absorbierenden Schicht der schmelzgeblasenen Schicht (41) eine weitere Schutzschicht (43) auf der Seite der absorbierenden Schicht (3) aufweist.

5. Absorbierender Artikel nach Anspruch 4, wobei der erhöhte Bereich (6) sowohl auf der Seite der nicht-absorbierenden Schicht als auch auf der Seite der absorbierenden Schicht angeordnet ist.

6. Absorbierender Artikel nach einem der Ansprüche 1 bis 5, wobei die Anzahl der Fasern, die von der schmelzgeblasenen Schicht (41) in die Schutzschicht (42) der Seite der nicht-absorbierenden Schicht eindringen vorzugsweise mindestens 2,5 Fasern/mm und höchstens 100 Fasern/mm beträgt, noch bevorzugter mindestens 5 Fasern/mm und höchstens 50 Fasern/mm und ferner bevorzugt mindestens 10 Fasern/mm und höchstens 40 Fasern/mm beträgt.

7. Absorbierender Artikel nach einem der Ansprüche 1 bis 6, wobei der erhöhte Bereich (6) oder der nicht-öffnende Bereich von dem Bereich, der mit der absorbierenden Schicht (3) überlappt, zu einem ebenen Bereich, der nicht mit der absorbierenden Schicht (3) außerhalb dieses Bereichs überlappt.

8. Absorbierender Artikel nach einem der Ansprüche 1 bis 7, wobei der durchschnittliche Faserdurchmesser der schmelzgeblasenen Schicht (41) vorzugsweise mindestens 0,1 μm und höchstens 2,5 μm, noch bevorzugter

mindestens 0,1 $\mu$m und höchstens 1,5 $\mu$m und ferner bevorzugt mindestens 0,1 $\mu$m und höchstens 1 $\mu$m beträgt.

9. Absorbierender Artikel nach einem der Ansprüche 1 bis 8, wobei ein Verhältnis des durchschnittlichen Faserdurchmessers der Fasern der schmelzgeblasenen Schicht (41) zu einem durchschnittlichen Faserdurchmesser der Fasern der Schutzschicht (42) vorzugsweise mindestens 1/100 und höchstens 1/8, noch bevorzugter mindestens 1/50 und höchstens 1/20 und ferner bevorzugt mindestens 1/40 und höchstens 1/25 beträgt.

10. Absorbierender Artikel nach einem der Ansprüche 1 bis 9, wobei ein Flächengewicht der schmelzgeblasenen Schicht (41) mindestens 3 g/m$^2$ und höchstens 20 g/m$^2$, noch bevorzugter 4 g/m$^2$ und höchstens 15 g/m$^2$ und ferner bevorzugt mindestens 5 g/m$^2$ und höchstens 10 g/m$^2$ beträgt.

11. Verfahren zur Herstellung eines Vliesstoffs zur Verwendung in einem absorbierenden Artikel nach Anspruch 1, wobei ultrafeine Fasern mit Hilfe eines Schmelzblasverfahrens auf einem Förderband angesammelt werden, um ein Rohmaterial der schmelzgeblasenen Schicht (41) in einer Maschinenrichtung zu befördern,

    eine Fläche des Rohmaterials der schmelzgeblasenen Schicht einer erhöhenden Behandlung unterzogen wird; und
    ein Rohmaterial einer Spinnvliesschicht auf eine der erhöhenden Behandlung unterzogenen Oberfläche des Rohmaterials der schmelzgeblasenen Schicht mit einem Spinnvliesverfahren laminiert wird.

## Revendications

1. Article absorbant comprenant une couche supérieure (1), une couche étanche (2), et une couche absorbante (3) disposée entre la couche supérieure (1) et la couche étanche (2) ;

    dans lequel la couche étanche (2) est directement empilée sur la couche absorbante (3),
    et la couche étanche (2) comprend une couche de fusion-soufflage (41) et une couche protectrice (42) de la couche obtenue par fusion-soufflage (41) ; et
    dans lequel la couche protectrice (42) est disposée sur un côté de couche non absorbante de la couche obtenue par fusion-soufflage (41) ;
    dans lequel la couche étanche (2) comprend une zone grattée (6), dans laquelle le nombre de fibres de la couche obtenue par fusion-soufflage (41) pour les fibres qui pénètrent dans la couche protectrice (42) est supérieur ou égal à 2,5 fibres/mm, le nombre de fibres étant mesuré avec un procédé tel que décrit dans la description ;
    dans lequel un diamètre moyen de fibre de fibres de la couche obtenue par fusion-soufflage (41) est inférieur ou égal à 2,5 $\mu$m ; et
    dans lequel la zone grattée (6) est disposée sur une zone dans laquelle la couche étanche (2) chevauche la couche absorbante (3).

2. Article absorbant selon la revendication 1,

    dans lequel la couche étanche (2) comprend une partie gaufrée dans laquelle la couche obtenue par fusion-soufflage (41) et la couche protectrice (42) sont liées, et une partie non gaufrée autre que la partie gaufrée ;
    dans lequel la couche étanche (2) comprend une zone sans ouverture dans la partie non gaufrée ;
    dans lequel la zone sans ouverture est une zone qui ne comprend pas de trou traversant dans lequel, dans une vue en plan de la couche étanche, un espace interfibre contenant une zone, dans laquelle une surface d'un rectangle formé de 10 $\mu$m dans une longueur de ligne diagonale atteint 25 $\mu$m$^2$ ou plus, passe à travers dans le sens de l'épaisseur, le trou traversant étant mesuré avec un procédé tel que décrit dans la description ; et
    dans lequel la zone sans ouverture est disposée sur une zone dans laquelle la couche étanche (2) chevauche la couche absorbante (3).

3. Article absorbant selon la revendication 1 ou 2, dans lequel la zone grattée (6) ou la zone sans ouverture est disposée sur une zone en tant qu'un tout, dans laquelle la couche étanche (2) chevauche la couche absorbante (3).

4. Article absorbant selon l'une quelconque des revendications 1 à 3, dans lequel la couche étanche (2) comprend une autre couche protectrice (43) sur le côté de couche absorbante (3) en plus de la couche protectrice (42) sur le côté de couche non absorbante de la couche obtenue par fusion-soufflage (41).

**5.** Article absorbant selon la revendication 4, dans lequel la zone grattée (6) est disposée sur le côté de couche absorbante en plus du côté de couche non absorbante.

**6.** Article absorbant selon l'une quelconque des revendications 1 à 5, dans lequel le nombre de fibres, qui pénètrent depuis la couche obtenue par fusion-soufflage (41) dans la couche protectrice (42) du côté de couche non absorbante est de préférence supérieur ou égal à 2,5 fibres/mm et est inférieur ou égal à 100 fibres/mm, de manière davantage préférée est supérieur ou égal à 5 fibres/mm et est inférieur ou égal à 50 fibres/mm, et est en outre de manière préférée supérieur ou égal à 10 fibres/mm et est inférieur ou égal à 40 fibres/mm.

**7.** Article absorbant selon l'une quelconque des revendications 1 à 6, dans lequel la zone grattée (6) ou la zone sans ouverture est disposée depuis la zone, qui chevauche la couche absorbante (3), à une zone plane, qui ne chevauche pas la couche absorbante (3) au-delà de cette zone.

**8.** Article absorbant selon l'une quelconque des revendications 1 à 7, dans lequel le diamètre de fibre moyen de fibres de la couche obtenue par fusion-soufflage (41) est de manière préférée supérieur ou égal à 0,1 $\mu$m et est inférieur ou égal à 2,5 $\mu$m, est de manière davantage préférée supérieur ou égal à 0,1 $\mu$m et est inférieur ou égal à 1,5 $\mu$m, et est en outre de manière préférée supérieur ou égal à 0,1 $\mu$m et est inférieur ou égal à 1 $\mu$m.

**9.** Article absorbant selon l'une quelconque des revendications 1 à 8, dans lequel un rapport du diamètre de fibre moyen de fibres de la couche de fusion-soufflage (41) sur un diamètre de fibre moyen de fibres de la couche protectrice (42) est de manière préférée supérieur ou égal à 1/100 et est inférieur ou égal à 1/8, est de manière davantage préférée supérieur ou égal à 1/50 et est inférieur ou égal à 1/20, et est en outre de manière préférée supérieur ou égal à 1/40 et est inférieur ou égal à 1/25.

**10.** Article absorbant selon l'une quelconque des revendications 1 à 9, dans lequel un poids de base de la couche de fusion-soufflage (41) est de manière préférée supérieur ou égal à 3 g/m$^2$ et est inférieur ou égal à 20 g/m$^2$, de manière davantage préférée est supérieur ou égal à 4 g/m$^2$ et est inférieur ou égal à 15 g/m$^2$, et est en outre de manière préférée supérieur ou égal à 5 g/m$^2$ et est inférieur ou égal à 10 g/m$^2$.

**11.** Procédé de production d'un tissu non tissé destiné à être utilisé dans un article absorbant selon la revendication 1, dans lequel des fibres ultrafines sont accumulées sur un convoyeur à bande par un procédé de fusion-soufflage pour convoyer en continu une matière première de couche obtenue par fusion-soufflage (41) dans un sens de machine ;

un traitement de grattage est appliqué à une surface de la matière première de couche de fusion-soufflage ; et une matière première de couche de filé-lié est stratifiée sur une surface traitée par grattage de la matière première de couche obtenue par fusion-soufflage par un procédé filé-lié.

{FIG. 1}

{FIG. 2}

{FIG. 3}

{FIG. 4}

{FIG. 5}

{FIG. 6}

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016065335 A
- JP 2011529749 T
- WO 2004094136 A1
- JP 2013013641 A